# EUROPEAN PATENT SPECIFICATION

(11) **EP 1 997 531 B1**
(45) Date of publication and mention of the grant of the patent: **26.12.2012**
(21) Application number: 07010871.7
(22) Date of filing: 01.06.2007
(51) Int. Cl.: A61N 5/10, G06F 19/00, G01T 1/161

(54) **Brachytherapy treatment system for effecting radiation treatment**
Brachytherapiebehandlungssystem zur Durchführung einer Strahlenbehandlung
Système de traitement en curiethérapie pour réaliser un traitement par rayonnement

(43) Date of publication of application: 03.12.2008
(73) Proprietor: Nucletron Operations B.V., 3905 TH Veenendaal (NL)
(72) Inventor: Kindlein, Johann, 47918 Toenisvorst (DE); Binnekamp, Dirk, 7623 CG Borne (NL)
(74) Representative: Jansen, Cornelis Marinus

(56) References cited:
- EP-A- 1 524 011
- WO-A-99/60921
- WO-A-2006/018307
- WO-A-2006/057911
- WO-A-2007/017847
- US-A- 6 055 450
- US-B1- 6 859 831

## Description

The invention relates to a brachytherapy treatment system for effecting radiation treatment on a pre-selected anatomical portion of an animal body, comprising
- first data processing means for generating a treatment plan for said pre-selected anatomical portion to be treated, partly based on image information of said pre-selected anatomical portion, wherein said treatment plan includes information concerning:
   * a number and an orientation of a plurality of hollow treatment channels to be inserted within said anatomical portion;
   * one or more positions and corresponding times of one or more radiation emitting sources to be inserted through said plurality of hollow treatment channels;
   * the amount of radiation dose to be emitted;
wherein the brachytherapy treatment system further comprises
- second data processing means for controlling at least one type of imaging means for generating image information of said pre-selected anatomical portion and said positions of said at least one of said hollow treatment channels within said anatomical portion and/or of said at least one energy emitting source within said hollow treatment channel, as well as treatment control means for controlling whether said positions of said at least one of said hollow treatment channels and/or of said at least one energy emitting source within said hollow treatment channel conform the pre-planned positions of said hollow treatment channels and/or of said at least one energy emitting source.

A brachytherapy system according to the above is for example disclosed in European patent application no. 1745820 in the name of the same applicant. A further brachytherapy system is disclosed in WO 99/60921. In the cancer treatment field, use is made of so-called remote after loaders to accurately advance and retract a flexible wire containing a gamma radiation emitting source over a specified distance for a specific time period. A remote after loader comprises a flexible simulation wire for testing purposes and a flexible wire with the gamma radiation emitting source, specific control and transport mechanisms to operate both types of wires, as well as a radiation shielded housing for the radiation emitting source.

The subsequent positioning of the energy emitting source through the interconnected delivery channels/treatment channels at several positions in the tumour is performed according to a predetermined sequence calculated by a treatment planning means in order to deliver a correct therapeutic dose of radiation to the tumour.

In order to avoid any misplacement of an energy emitting source at the wrong position or in the wrong treatment channel, control information is to be obtained in real time and to be fed back towards the first processing means for generating the treatment plan for evaluation purposes and - in the event of any deviation between the pre-planned information and the actual information obtained in real time - for generating a new treatment plan using the feedback information in order to deliver the prescribed therapeutic dose of radiation.

The present brachytherapy systems lack a proper control feedback in real time from the second data processing means to the first data processing means concerning the actual position of the body and the treated organ as well of the critical organs and the actual orientation and position of the treatment channels inserted into the patient's body and the actual position of the energy emitting sources inside each treatment channel. Moreover obtaining, handling and processing in real time the actual status information concerning the treatment being performed sets a heavy burden on its control resources such as computing time, signal processing capacity and computing time/speed.

According the invention, the brachytherapy treatment system is characterized in that said brachytherapy system comprises at least one processor based operating system arranged in operating during use said first data processing means on a non-real time basis and operating said second data processing means on a real time basis and, in the event said second processing means are being operated, said system is further being arranged in appointing said processor computing time for a specific computing period solely to the second processing means, allowing the second processing means to control the first processing means in real time.

With the features of the invention a more versatile brachytherapy system is obtained since the system functions which require a 'real time' operation are given a higher priority by the control system rather than the system functions which require a 'non-real time' operation. The system resources (like memory usage and processor calculation time) are better managed, allowing the brachytherapy treatment system to be used in an adaptive manner, meaning that the system is capable of handling and managing a treatment session to be performed, in particular when the course of treatment needs to be altered rather quickly and in real time due to (unforeseen) changing circumstances (like incorrect placement of hollow treatment channels and/or energy emitting sources, a non-conformal radiation dose being emitted in view of the dose being pre-planned, etc.).

In a specific embodiment, the brachytherapy treatment system according to the invention is characterized in that the system is a single processor based system, whereas in another functional embodiment, the brachytherapy treatment system is provided with at least one multi-processor system.

In a further improved embodiment, the brachytherapy treatment system is arranged in managing certain system functions with a higher priority and hence preferably in a real time control environment using the real time operated second control means. For example, said brachytherapy therapy system may comprise a treatment table for positioning said animal body, wherein said in real time operated second processing means further comprises treatment table control means.

Also said brachytherapy therapy system may comprise channel tracking means for tracking the insertion of at least one of said plurality of hollow treatment channel into said anatomical portion, wherein said in real time operated second processing means further comprises channel tracking control means.

Also according to a further embodiment, said brachytherapy treatment system may comprise source tracking means for tracking said at least one energy emitting source within said hollow treatment channel into said anatomical portion, wherein said in real time operated second processing means further comprises source tracking control means.

The aforementioned features require a real time control environment or functionality and hence require control at a higher priority level using the second control means.

In order to allow and obtain an accurate tracking of the actual position of the energy emitting source whilst being inserted into a patient's body, the source tracking means comprise at least two energy detecting sensors being movable with respect to each other for determining the position of at least one energy emitting source in real time.

Preferably, the detecting sensors are surface detecting sensors provided with energy collimating elements having a plurality of parallel extending linear bores, wherein furthermore the detecting sensor and the collimator element are linear shaped. This allows the detection of radiation from a specific direction, thus further improving tracking of the actual position of the source.

In view of the above preferred characteristics of the energy collimating element is collimating element is made from a material having a high atomic number Z, with preferably Z>50.

In a further embodiment, tracking of the position of the source is improved due to the fact that each energy detecting sensor detects the direction from which one energy emitting source is emitting towards said energy detecting sensor.

In particular, said source tracking control means are arranged in determining in real time the actual position of said energy emitting source based on the intersection of two directions being detected by two energy detecting sensors. This allows a quick yet accurate detection of each energy emitting source (in the 3D space) inserted into a patient's body, thus improving the quality of the treatment being performed.

The invention will now be further explained using the following figures:
Figure 1 shows a brachytherapy treatment system according to the state of the art;
Figure 2 shows a first embodiment of a brachytherapy treatment system according to the invention;
Figures 3-5 show further embodiments of a brachytherapy treatment system according to the invention.

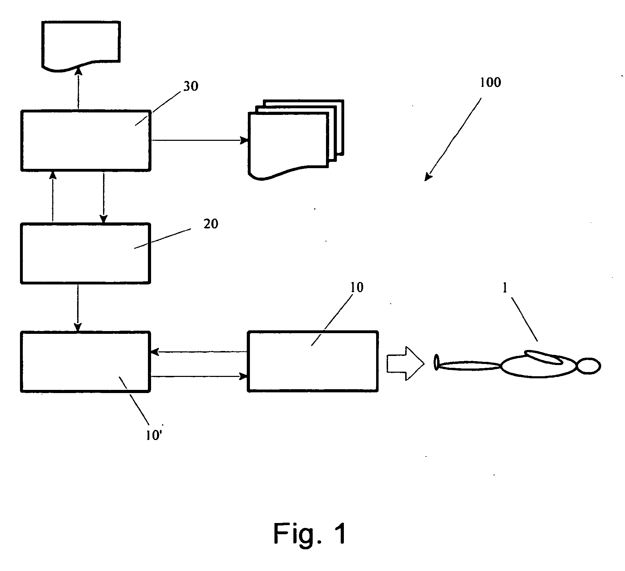

In Figure 1, a schematic view of a brachytherapy treatment system according to the state of the art is shown.

Reference numeral 1 depicts a patient lying in a treatment position during a brachytherapy treatment session using an afterloading device, for example disclosed in the applicant's pending European patent application no. 1745820. Reference numeral 10 denotes an afterloader apparatus as well as its treatment control means for controlling the afterloader apparatus for inserting one or more hollow treatment needles into the patient's body 1 towards an intended site (e.g. a cancerous tumour) to be treated with energy/radiation emitted by energy emitting sources, which are subsequently inserted from the afterloader apparatus 10 through the implanted hollow treatment channel.

The afterloader apparatus/treatment control means 10 are controlled by processing means 10' which in turn receives treatment planning information from treatment planning means 20. A known example of such treatment planning means 20 is the planning software PLATO^{™} from Nucletron B.V. which generate a radiation treatment plan for said patient 1 partly based on image information obtained from the pre-selected anatomical portion/tumour to be treated. The treatment plan generated by the treatment planning means 20 contains information concerning a desired number and orientation of hollow treatment channels to be inserted within the patient's body 1; one or more orientation/positions and corresponding dwell times of one or more energy emitting sources to be inserted through said hollow treatment channels until within the patient's body 1, as well as the amount of radiation dose to be emitted by said energy emitting sources.

Usually, the treatment planning means 20 is a client system connected in a network environment with a treatment plan server 30 on which important patient data (of several patients) are stored. The whole brachytherapy treatment proces is controlled and managed by the processing means 10'.

In order to allow an actual, real-time monitoring of the course of the treatment session to be performed, a significant amount of status information is to be obtained in real-time and to be handled and evaluated in real-time by the processing means 10' of the brachytherapy treatment system.

To this end and according to the invention, the processing means 10' of the brachytherapy treatment system 100' are composed of first data processing means 10b', second data processing means 10a' as well as at least one processor based operating system 10c'. The processor based operating system 10c' is arranged in operating, during use, said first data processing means 10b' on a non-real-time basis. Moreover, the processor based operating system 10c' is arranged in operating during use said second data processing means 10a' on a real-time basis.

The advantage of this system configuration lies in the fact that in the event that the second data processing means are being operated, the operating system 10c' appoints processor computing time during a specific computing period solely to the second processing means 10a'.

In this operational situation, the second processing means are allowed to operate the brachytherapy treatment system 100' in a "real-time" mode, thus giving a higher priority to the system functions which require a real-time operation rather than to the system functions which require a "non-real-time" operation.

Also according to the invention, the second data processing means 10a' are arranged in controlling the first data processing means 10b'. The brachytherapy treatment system 100' is allowed to be used in an adaptive manner. Especially with the second data processing means 10a' being given a higher priority in view of the usage of system resources (memory, processor processing time), the system is more capable of handling and managing a treatment session to be performed in real-time, in particular when the course of treatment needs to be altered rather quickly and in real-time due to unforseen changing circumstances, like incorrect placement of hollow treatment channels and/or energy emitting sources.

All system resources which do not require a real-time operation are set to a lower priority and are only then "re-instated" towards a normal priority by the second data processing means 10a' when the operation of the brachytherapy treatment system in real-time and at a high priority is interrupted or no longer (or to a lesser extent) needed.

In view of the improved functionality of the brachytherapy treatment system according to the invention, the second data processing means 10a' are appointed a higher priority in terms of processing computing time for a specific computing period allowing a more accurate operation in real-time of the complete system (and components) especially when performing specific system functions which require or prefer an accurate real-time operation.

Preferably, the operating system 10c' which is based on at least one processor module can be regarded as a single processor based operating system. However, also a multi-processor based operating system 10c' can be implemented, resulting in a more versatile brachytherapy treatment system in view of the real-time obtaining, handling and processing of status information concerning the therapy treatment session to be performed.

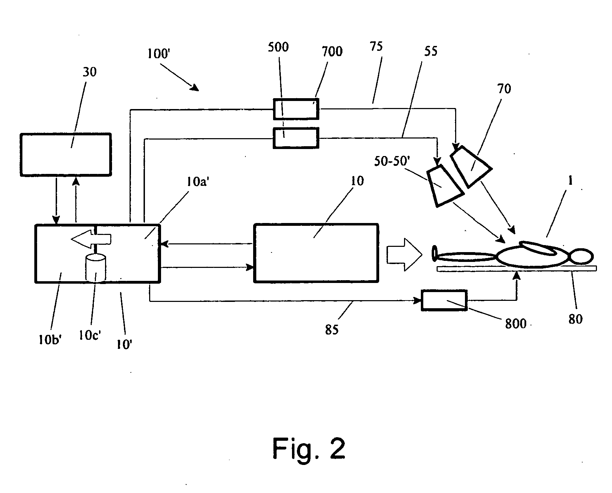

For example and as also depicted in Figure 2, the second data processing means 10a' may operate treatment table control means 800 which in turn control (via signal line 85) a treatment table 80 on which the patient's body 1 is positioned during the course of the radiation treatment. This allows in real-time a quick and accurate (re)positioning of the patient 1 relative to the afterloading apparatus 10 or imaging means used to obtain image information of the internal organs/tumour to be treated.

Likewise, the brachytherapy treatment system 100' may comprise channel tracking means 70 being controlled by channel tracking control means 700 which in turn are operated and controlled by the second data processing means 10a' via signal line 75. The channel tracking means 70 shown in Figure 2 are used for tracking in real-time the insertion of at least one of said plurality of hollow treatment channels into the patient's body 1. In fact, whilst operating the channel tracking means 70/700 in real-time, a prompt, accurate and complete overview about the actual position of the treatment channel during/after insertion in the patient's body 1 is obtained, and the brachytherapy treatment can be adapted in the event that any deviation of the course of treatment occurs.

Thus, any deviation from the planned/intended treatment parameters can be registered, evaluated and compared with the treatment planning data, and, in case of deviations which are outside of an allowed window the user is alerted and requested to decide about the interruption or continuation of the treatment course. If the treatment has been interrupted, the corresponding changes which have been the cause of the interruption are used by the processing means 10b' for correction.

Likewise, the brachytherapy treatment system 100' may comprise source tracking means 50-50' which are controlled by source tracking control means 500 controlled in real-time via signal line 55 by the second data processing means 10a'.

Similar to the channel tracking means 70-700, as discussed above, also the source tracking means 50-50'-500 are used to track or follow in real-time the actual position of each energy emitting source being inserted through the implanted treatment channels into the patient's body 1. In fact, with the source tracking means 50-50'-500, it is possible to trace in real-time the actual position of each energy emitting source within the patient's body 1, thus allowing a direct and unambiguous evaluation with the corresponding planned parameters generated by the first data processing means 10b'.

Hence, with this embodiment, a real-time tracking system for an energy emitting source implanted in a patient's body 1 is obtained.

With this embodiment and in particular with the second data processing means 10b' being operated on a real-time basis and having a higher priority or a majority of the available processor computing time of the operating system 10c', an accurate and precise reconstruction of the actual radiation dose distribution being emitted to the tumour to be treated is obtained. When reconstructing the actual radiation dose distribution being emitted, the actual, real-time information concerning the patient's geometry and the actual position of the (different) energy emitting sources being implanted is required. This information can be obtained accurately and in real-time, when the second data processing means 10b' are given a higher priority over the first data processing means 10b' in view of processor access, processor computing time and usage of other computing resources, such as usage of memory.

For a proper and accurate and more in particular a real-time reconstruction of the radiation dose actually delivered during the course of treatment, an accurate detection system is required for obtaining accurate information about the exact position of each energy emitting source within each treatment channel being implanted in the patient's body in real-time. Apart from the usage of additional imaging means which generate in real-time accurate image information about the patient's body 1, also an accurate localisation of the energy emitting sources being implanted is required.

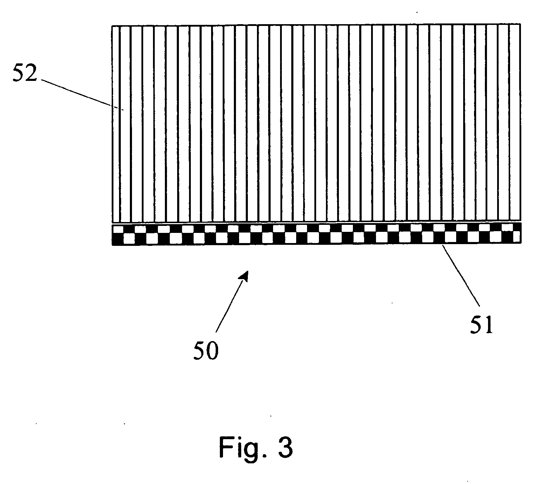

An embodiment of a measuring system for accurately detecting the actual, real-time location of an energy emitting source implanted in a patient's body 1 is shown in Figures 3-5. With this detection system, the exact location of an energy emitting point-like source is obtained using only two special constructed radiation detectors 50-50', one of the sensors being depicted in Figure 3.

In Figure 3, the reference numeral 50 denotes a sensor provided with an energy sensitive detecting surface 51 and an energy collimating element 52. The energy collimating element 52 is provided as a block-shaped element with a plurality of parallel linear bores. These bores serve as collimating channels and only energy or radiation being emitted by an energy emitting source is detected by the detecting surface 51 in the event that the path of the incidents energy/radiation being detected is substantially parallel to the linear bores extending parallel to each other.

Although the energy sensitive detecting surface 51 may have a two-dimensional construction, in another embodiment the two-dimensional surface-arrangement of the detecting sensor 50 and the energy collimating element 52 can be reduced to a (straight) line.

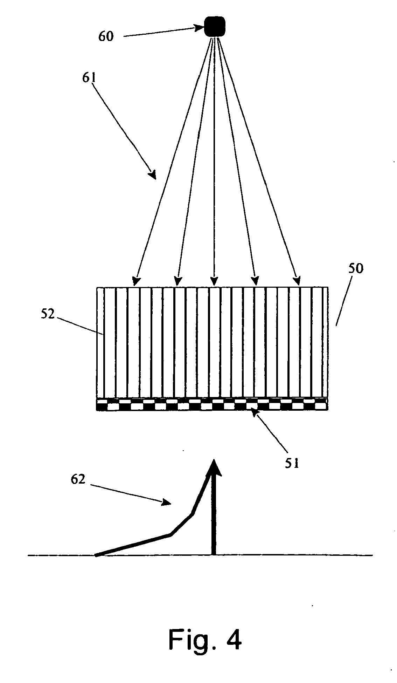

This is shown in Figure 4, wherein the reference number 62 denotes an energy detecting profile showing the amount of energy being detected by the sensor 50 depending on the angle of incidence of the radiation 61 being emitted by the point-shaped energy emitting source 60. Only or almost only radiation being emitted by the energy emitting source 60 in a parallel orientation to the multiple bores, is detected.

In order to obtain an accurate orientation/location of an energy emitting source being advanced through a hollow treatment channel towards an intended position within a patient's embody 1 the source tracking means 50-50'-500 are positioned in the propagation path A-A'; B-B' of the radiation being emitted by the energy emitting source positioned at two subsequent positions along the displacement path S(X,Y,Z).

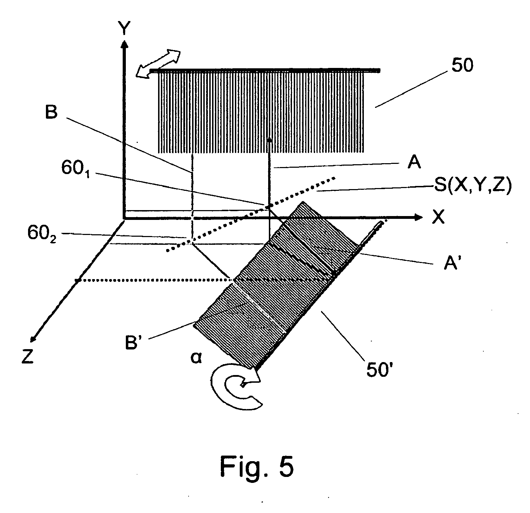

In Figure 5, the two subsequent positions of the energy emitting source to be localized are denoted with reference numbers 60₁-60₂.

The two sensors 50-50' detect energy/radiations being emitted by the energy emitting source 60₁-60₂ from two directions. By a proper analysis of the corresponding radiation emission profiles 62 (see Figure 4) detected by each sensor 50-50', the actual propagation path A-A' of the radiation being emitted can be reconstructed. The actual position of the energy emitting source is determined by the intersection of both propagation paths A-A' and is denoted with the reference number 60₁.

As the energy emitting source 60, is advanced along a certain trajectory through a treatment channel within the patient's body 1, a subsequent position of the energy emitting source (reference number 60₂) can be obtained by repositioning both sensors 50-50' in the X-Y-Z-space, meaning the displacement of both or one sensor 50-50' in a transverse orientation or by its rotation by an angle α.

Again the corresponding radiation emission profiles 62 are detected and analysed by the source tracking means 500, and the peak signal will define the propagation path B-B' of the majority of the radiation being emitted by the source in said position 60₂. Again the intersection between both imaginary lines B-B' will define the actual position of the energy emitting source 60₂ being advanced along the line defined by S(X,Y,Z).

Preferably, the collimating elements 52 are made of a material that effectively absorbs incident and secondary radiation rays 61 being emitted by an energy emitting source 60. A material having a high atomic number (having Z greater than 50) appears to be an effective absorber of radiation impinging on the sensor surface 51, at an angle α.

## Claims

1. Brachytherapy planning system (100) for effecting radiation treatment on a pre-selected anatomical portion of an animal body, comprising:
- first data processing means (10b) for generating a treatment plan for said pre-selected anatomical portion to be treated, partly based on image information of said pre-selected anatomical portion, wherein said treatment plan includes information concerning:
- a number and an orientation of a plurality of hollow treatment channels to be inserted within said anatomical portion;
- one or more positions and corresponding times of at least one energy emitting source to be inserted through said plurality of hollow treatment channels;
- the amount of radiation dose to be deposited;
wherein the brachytherapy treatment system further comprises
- second data processing means (10a) for controlling at least one type of imaging means for generating image information of said pre-selected anatomical portion and said positions of said at least one of said hollow treatment channels within said anatomical portion and/or of said at least one energy emitting source within said hollow treatment channel, as well as treatment control means for controlling whether said positions of said at least one of said hollow treatment channels and/or of said at least one energy emitting source within said hollow treatment channel conform the pre-planned positions of said hollow treatment channels and/or said at least one energy emitting source,
**characterized in that**
said brachytherapy system (100) comprises at least one processor based operating system (10c) arranged in operating during use said first data processing means (10b) on a non real time basis and operating said second data processing means (10a) on a real-time basis in the event said second data processing means (10a) are being operated, said operating system further being arranged in appointing said processor computing time for a specific computing period solely to the second data processing means (10a), thus allowing the second data processing means (10a) to control the first data processing means (10b) in real-time, wherein, in the event of any deviation between the pre-planned information and the actual information obtained in real time, the first processing means is configured to generate a new treatment plan in order to alter the course of treatment in real time.

2. Brachytherapy treatment system (100') according to claim 1, **characterized in that** the first as well as the second processing means (10b' 10a') are single core processor means running one or more real time and non real-time operating systems.

3. Brachytherapy treatment system (100') according to claim 1, **characterized in that** the first as well as the second processing means 110b', 10a') are multi core processor means, wherein real time and non real time processes are running on independent processor cores, and wherein at least one operating system is a multi-processor based operating system.

4. Brachytherapy treatment system (100') according to any of the preceding claims, **characterized in that** said brachytherapy treatment system comprises a treatment table for positioning said animal body, and wherein said second processing means (10a') operated in real time further comprises treatment table control means.

5. Brachytherapy treatment system (100') according to any of the preceding claims, **characterized in that** said brachytherapy treatment system comprises channel tracking means (70) for tracking the insertion of at least one of said plurality of hollow treatment channels into said anatomical portion, and wherein said second processing means (10a') operated in real time further comprises channel tracking control means (700).

6. Brachytherapy treatment system according to any of the preceding claims, **characterized in that** said brachytherapy treatment system comprises source tracking means (50-50') for tracking said at least one energy emitting source within the respective hollow treatment channel into said anatomical portion, and wherein said second processing means operated in real time further comprises source tracking control means.

7. Brachytherapy treatment system according to claim 6, **characterized in that** the source tracking means comprise at least two energy detecting sensors being movable with respect to each other for determining the position of the at least one energy emitting source in real time.

8. Brachytherapy treatment system (100') according to claim 7, **characterized in that** the energy detecting sensors are surface detecting sensors provided with energy collimating elements having a plurality of parallel linear bores (52).

9. Brachytherapy treatment system according to claim 8, **characterized in that** the energy detecting sensor and the collimating element are linear shaped.

10. Brachytherapy treatment system according to claim 8 or 9, **characterized in that** said energy collimating element is made from a material having an atomic number Z of at least 50.

11. Brachytherapy treatment system according to any of the claim 7-10, **characterized in that** each energy detecting sensor is configured to detect the direction from which one energy emitting source is emitting radiation towards said energy emitting sensor.

12. Brachytherapy treatment system according to claim 11, **characterized in that** said source tracking control means are configured to determine the actual position of said energy emitting source in real time based on the intersection of two directions being detected by said at least two energy detecting sensors.

## Patentansprüche

1. Brachytherapieplanungssystem (100) zur Durchführung einer Strahlenbehandlung an einem vorbestimmten anatomischen Teil eines Tierkörpers, umfassend:
- erste Datenverarbeitungsmittel (10b) zum Erzeugen eines Behandlungsplans für den zu behandelnden vorbestimmten anatomischen Teil, teilweise basierend auf Bildinformation des vorbestimmten anatomischen Teils, wobei der Behandlungsplan Informationen umfasst, die Folgendes betreffen:
- eine Anzahl und eine Ausrichtung von mehreren, in den anatomischen Teil einzuführenden hohlen Behandlungskanälen;
- eine oder mehrere Positionen und entsprechende Zeiten von mindestens einer energieemittierenden Quelle, die durch die mehreren hohlen Behandlungskanäle einzuführen ist;
- die Menge abzugebender Strahlungsdosen;
wobei das Brachytheraphiebehandlungssystem ferner umfasst:
- zweite Datenverarbeitungsmittel (10a) zum Steuern von mindestens einem Typ von Bildverarbeitungsmitteln zum Erzeugen von Bildinformation des vorbestimmten anatomischen Teils und der Positionen von mindestens einem der hohlen Behandlungskanäle in dem anatomischen Teil und/oder von der mindestens einen energieemittierenden Quelle in dem hohlen Behandlungskanal, sowie Behandlungssteuerungsmittel, um zu steuern, ob die Positionen des mindestens einen der hohlen Behandlungskanäle und/oder der mindestens einen energieemittierenden Quelle in dem hohlen Behandlungskanal konform der vorgeplanten Positionen der hohlen Behandlungskanäle und/oder der mindestens einen energieemittierenden Quelle ist,
**dadurch gekennzeichnet, dass**
das Brachytherapiesystem (100) mindestens ein prozessorbasierendes Betriebssystem (10c) umfasst, das angeordnet ist, um während der Verwendung die ersten Datenverarbeitungsmittel (10b) auf Nicht-Echtzeitbasis zu betreiben und die zweiten Datenverarbeitungsmittel (10a) auf Echtzeitbasis zu betreiben, falls die zweiten Datenverarbeitungsmittel (10a) betrieben werden, wobei das Betriebssystem ferner angeordnet ist, um die Prozessorrechenzeit für eine bestimmte Rechenperiode ausschließlich den zweiten Datenverarbeitungsmitteln (10a) zuzuweisen und es so den zweiten Datenverarbeitungsmitteln (10a) zu erlauben, die ersten Datenverarbeitungsmittel (10b) in Echtzeit zu steuern, wobei im Fall irgendeiner Abweichung zwischen der vorgeplanten Information und der effektiven, in Echtzeit erhaltenen Information die ersten Verarbeitungsmittel konfiguriert sind, um einen neuen Behandlungsplan zu erzeugen, um den Behandlungsverlauf in Echtzeit zu ändern.

2. Brachytherapiebehandlungssystem (100') nach Anspruch 1, **dadurch gekennzeichnet, dass** die ersten wie auch die zweiten Verarbeitungsmittel (10b', 10a') Mehrkernprozessormittel sind, die ein oder mehrere Echtzeit- und Nicht-Echtzeit-Betriebssysteme ausführen.

3. Brachytherapiebehandlungssytem (100') nach Anspruch 1, **dadurch gekennzeichnet, dass** die ersten wie auch die zweiten Verarbeitungsmittel (10b', 10a') Mehrkernprozessormittel sind, wobei Echtzeit- und Nicht-Echtzeit-Prozesse auf unabhängigen Prozessorkernen ausgeführt werden, und wobei mindestens ein Betriebssystem ein Betriebssystem basierend auf mehreren Prozessoren ist.

4. Brachytherapiebehandlungssystem (100') nach einem der vorhergehenden Ansprüche, **dadurch gekennzeichnet, dass** das Brachytherapiebehandlungssystem einen Behandlungstisch zum Positionieren des Tierkörpers umfasst und wobei die zweiten in Echtzeit betriebenen Verarbeitungsmittel (10a') ferner Mittel zum Steuern des Behandlungstisches umfassen.

5. Brachytherapiebehandlungssystem (100') nach einem der vorhergehenden Ansprüche, **dadurch gekennzeichnet, dass** das Brachytherapiebehandlungssystem Kanalverfolgungsmittel (70) umfasst, um das Einführen des mindestens einen der mehreren hohlen Behandlungskanäle in den anatomischen Teil zu verfolgen, und wobei die zweiten in Echtzeit betriebenen Verarbeitungsmittel (10a') ferner Kanalverfolgungssteuermittel (700) umfassen.

6. Brachytherapiebehandlungssystem nach einem der vorhergehenden Ansprüche, **dadurch gekennzeichnet, dass** das Brachytherapiebehandlungssystem Quellverfolgungsmittel (50-50') umfasst, um die mindestens eine energieemittierende Quelle in dem jeweiligen hohlen Behandlungskanal in dem anatomischen Teil zu verfolgen, und wobei die zweiten in Echtzeit betriebenen Verarbeitungsmittel ferner Quellverfolgungssteuermittel umfassen.

7. Brachytherapiebehandlungssystem nach Anspruch 6, **dadurch gekennzeichnet, dass** die Quellverfolgungsmittel mindestens zwei zueinander bewegliche energiedetektierende Sensoren umfassen, um die Position der mindestens einen energieemittierenden Quelle in Echtzeit zu bestimmen.

8. Brachytherapiebehandlungssystem (100') nach Anspruch 7, **dadurch gekennzeichnet, dass** die energiedetektierenden Sensoren oberflächendetektierende Sensoren sind, versehen mit energiekollimatierenden Elementen mit mehreren parallelen linearen Bohrungen (52).

9. Brachytherapiebehandlungssystem nach Anspruch 8, **dadurch gekennzeichnet, dass** der energiedetektierende Sensor und das kollimatierende Element linear geformt sind.

10. Brachytherapiebehandlungssystem nach Anspruch 8 oder 9, **dadurch gekennzeichnet, dass** das energiekollimatierende Element aus einem Material mit einer Atomzahl Z von mindestens 50 hergestellt ist.

11. Brachytherapiebehandlungssystem nach einem der Ansprüche 7-10, **dadurch gekennzeichnet, dass** jeder energiedetektierende Sensor konfiguriert ist, um die Richtung zu detektieren, aus der eine energieemittierende Quelle Strahlung zu dem energieemittierenden Sensor emittiert.

12. Brachytherapiebehandlungssystem nach Anspruch 11, **dadurch gekennzeichnet, dass** die Quellverfolgungssteuermittel konfiguriert sind, um in Echtzeit die aktuelle Position der energieemittierenden Quelle basierend auf dem Schnittpunkt zweier Richtungen zu bestimmen, die von den mindestens zwei energiedetektierenden Sensoren detektiert werden.

## Revendications

1. Système de planification de curiethérapie (100) pour effectuer un traitement par rayonnement sur une partie anatomique présélectionnée du corps d'un animal, comprenant :
- un premier moyen de traitement de données (10b) servant à générer pour ladite partie anatomique présélectionnée à traiter un plan de traitement en partie basé sur des informations d'images de ladite partie anatomique présélectionnée, lequel plan de traitement contient des informations concernant :
- un nombre et une orientation d'une pluralité de conduits de traitement creux à introduire dans ladite partie anatomique ;
- une ou plusieurs positions, et un ou plusieurs temps correspondants, d'au moins une source émettrice d'énergie à introduire par le biais de ladite pluralité de conduits de traitement creux ;
- la quantité de la dose de rayonnement à délivrer ;
dans lequel le système de traitement par curiethérapie comprend en outre :
- un deuxième moyen de traitement de données (10a) pour commander au moins un type de moyen d'imagerie servant à générer des informations d'images de ladite partie anatomique présélectionnée et desdites positions de l'un au moins desdits conduits de traitement creux à l'intérieur de ladite partie anatomique et/ou de ladite au moins une source émettrice d'énergie à l'intérieur dudit conduit de traitement creux, ainsi qu'un moyen de contrôle de traitement pour contrôler si lesdites positions de l'un au moins desdits conduits de traitement creux et/ou de ladite au moins une source émettrice d'énergie à l'intérieur dudit conduit de traitement creux sont conformes aux positions pré-planifiées desdits conduits de traitement creux et/ou de ladite au moins une source émettrice d'énergie,
**caractérisé en ce que**
ledit système de curiethérapie (100) comprend au moins un système d'exploitation (10c), tournant sur un processeur, adapté pour, à l'utilisation, faire fonctionner ledit premier moyen de traitement de données (10b) selon un mode non en temps réel et pour faire fonctionner ledit deuxième moyen de traitement de données (10a) selon un mode en temps réel dans le cas où ledit deuxième moyen de traitement de données (10a) est en fonctionnement, ledit système d'exploitation étant en outre adapté pour que ledit processor alloue du temps de calcul pendant une durée de calcul spécifique uniquement au deuxième moyen de traitement de données (10a), permettant ainsi au deuxième moyen de traitement de données (10a) de contrôler le premier moyen de traitement de données (10b) en temps réel, dans lequel, en cas d'écart entre les informations pré-planifiées et les informations concrètes obtenues en temps réel, le premier moyen de traitement est configuré pour générer un nouveau plan de traitement afin de modifier le cours du traitement en temps réel.

2. Système de traitement par curiethérapie (100') selon la revendication 1, **caractérisé en ce que** le premier ainsi que le deuxième moyen de traitement (10b', 10a') sont des moyens de processeurs simple coeur sur lesquels tournent un ou plusieurs systèmes d'exploitation en temps réel et non en temps réel.

3. Système de traitement par curiethérapie (100') selon la revendication 1, **caractérisé en ce que** le premier ainsi que le deuxième moyen de traitement (10b', 10a') sont des moyens de processeurs multicoeurs, des processus en temps réel et non en temps réel étant exécutés sur des coeurs de processeurs indépendants et au moins un système d'exploitation étant un système d'exploitation multiple tournant sur un processeur.

4. Système de traitement par curiethérapie (100') selon l'une quelconque des revendications précédentes, **caractérisé en ce que** ledit système de traitement par curiethérapie comprend une table de traitement servant à positionner ledit corps d'animal et ledit deuxième moyen de traitement (10a') fonctionnant en temps réel comprend en outre un moyen de commande de la table de traitement.

5. Système de traitement par curiethérapie (100') selon l'une quelconque des revendications précédentes, **caractérisé en ce que** ledit système de traitement par curiethérapie comprend un moyen de localisation de conduit (70) pour suivre l'insertion de l'un au moins de ladite pluralité de conduits de traitement creux dans ladite partie anatomique et ledit deuxième moyen de traitement (10a1) fonctionnant en temps réel comprend en outre un moyen de commande de localisation de conduit (700).

6. Système de traitement par curiethérapie selon l'une quelconque des revendications précédentes, **caractérisé en ce que** ledit système de traitement par curiethérapie comprend des moyens de localisation de source (50, 50') pour suivre ladite au moins une source émettrice d'énergie à l'intérieur du conduit de traitement creux respectif dans ladite partie anatomique et ledit deuxième moyen de traitement fonctionnant en temps réel comprend en outre un moyen de commande de localisation de source.

7. Système de traitement par curiethérapie selon la revendication 6, **caractérisé en ce que** les moyens de localisation de source comprennent au moins deux capteurs de détection d'énergie mobiles les uns par rapport aux autres afin de déterminer la position de la ou des sources émettrices d'énergie en temps réel.

8. Système de traitement par curiethérapie (100') selon la revendication 7, **caractérisé en ce que** les capteurs de détection d'énergie sont des capteurs de détection de surface dotés d'éléments de collimation d'énergie qui possèdent une pluralité de perçages linéaires parallèles (52).

9. Système de traitement par curiethérapie selon la revendication 8, **caractérisé en ce que** le capteur de détection d'énergie et l'élément de collimation sont de forme linéaire.

10. Système de traitement par curiethérapie selon la revendication 8 ou la revendication 9, **caractérisé en ce que** ledit élément de collimation d'énergie est fait d'un matériau dont le numéro atomique Z est au moins égal à 50.

11. Système de traitement par curiethérapie selon l'une quelconque des revendications 7 à 10, **caractérisé en ce que** chaque capteur de détection d'énergie est configuré pour détecter la direction à partir de laquelle une source émettrice d'énergie émet un rayonnement en direction dudit capteur de détection d'énergie.

12. Système de traitement par curiethérapie selon la revendication 11, **caractérisé en ce que** ledit moyen de commande de localisation de source est configuré pour déterminer la position réelle de ladite source émettrice d'énergie en temps réel sur la base de l'intersection de deux directions détectées par lesdits au moins deux capteurs de détection d'énergie.
